# EUROPEAN PATENT SPECIFICATION

(11) **EP 2 395 948 B1**
(45) Date of publication and mention of the grant of the patent: **28.11.2012**
(21) Application number: 10711731.9
(22) Date of filing: 12.02.2010
(51) Int. Cl.: A61F 2/44, A61F 2/46

(54) **INTERVERTEBRAL DISC PROSTHESIS FOR STABILIZING THE LUMBAR SPINE AND RELEVANT KIT**
BANDSCHEIBENPROTHESE ZUM STABILISIEREN DER LENDENWIRBELSÄULE UND ENTSPRECHENDES KIT
PROTHÈSE DE DISQUE INTERVERTÉBRAL DESTINÉE À STABILISER LA COLONNE LOMBAIRE, ET KIT AFFÉRENT

(30) Priority: 13.02.2009 IT RM20090065
(43) Date of publication of application: 21.12.2011
(73) Proprietor: 2B1 S.r.l., 20124 Milano (IT)
(72) Inventor: MIGLIETTA, Carlo, I-20124 Milano (IT); INVERNIZZI, Gloria, I-20124 Milano (IT); MARROCU, Alessandro, I-20124 Milano (IT)
(74) Representative: Santi, Filippo
(86) International application number: PCT/IT2010/000048
(87) International publication number: WO 2010/092613

(56) References cited:
- US-A- 6 126 689
- US-A1- 2005 021 144
- US-A1- 2007 073 398

## Description

The present invention concerns intervertebral disc prosthesis for stabilization of lumbar spine and relevant kit.

More specifically the invention relates to prosthesis of the above kind for correction of spine that can restore the physiological movement range of two vertebral bodies of the spine.

In the following, the specification will be mainly addressed to the correction of the lumbar portion of the spine, but it is well evident that the same must not be considered limited to this specific use.

As it is well known, spine is often subjected to different pathologies. In many cases, it is also necessary replacing the intervertebral disco, and particularly both outer annulus and pulpous nucleus.

Lumbago, of lumbar pain, is among pathologies very often requiring replacement of intervertebral disco by implanting prosthesis. Such pathology very frequently occurs and can cause strong pains to the suffering person. Prosthesis replacement surgical intervention is usually carried out in case of chronic lumbago not responding to preservative or pharmacological treatments.

At present, a huge number of intervertebral disc prosthesis is available on the market that however suffers of different problems.

First limit of known prosthesis is that they tend to be very rigid under a mechanical point of view. Particularly, said prosthesis, just for the position where they should be placed, are subjected to remarkable mechanical stresses, caused by numerous flexion, rotation and torsion movements toy which human spins is subjected. Intervertebral disco prosthesis presently available on the market are too rigid and often do not permit to the subject on which they have been implanted recovering all the possible movements.

In fact, under a strictly functional point of view, it has been observed that known prosthesis have a reduced level of reactivation of lordotico alignment, not being able maintaining physiological movement range for flexion/extension, rotation and lateral inclination, with the consequent remarkable limitation of movement for the patient.

A second problem of known prosthesis, which is much more serious than the first one, is that they usually are bulky. This implies that it is not possible placing them between vertebrae, by a well known and not invasive discectomy surgical intervention, reaching the site where said prosthesis should be implanted from behind the patient, as it would be more natural and less invasive. Moreover, as far as cinematic aspects are concerned, at present technology of disco prosthesis replacement has two main basic approaches to the freedom degree permitted with respect to the mono-axial rotation:
a. unconstrained devices, with a variable rotation centre permitting segmental rotation and translation along three axes X, Y, Z;
b. constrained devices, with fixed rotation centre, so that anteroposterior and lateral translation movements are prevented, while rotation movements are permitted.

Unconstrained devices require presence of an intact structure of ligaments. Instead, constrained devices do not ensure restoring of all movements permitted by natural disco,

Prosthesis presently available must be implanted by a surgical intervention, entering from abdomen. This clearly implies different intervention and post- intervention complications, besides higher risks, among which:
- stresses of vital organs and parts;
- a higher difficulty for surgeon, translating into a reduction of probabilities of success or the intervention;
- a lower number of patients that can be subjected to the intervention, for example due to age, health and like.

As a latter aspect to be taken into consideration, but not less important, known prosthesis are very expensive.

The relevant prior art includes the patent applications US 2005/021144A1 (henceforth D1), US 2007/073398A1 (henceforth D2) and US 61266689A.

In view of the above, it is therefore specific object of the present invention that of suggesting an intervertebral disco prosthesis for stabilization of spine, particularly of lumbar tract, that can be installed from behind between two vertebral bodies by a simple discectomy (so called mini-invasive access), following well consolidated known surgical technical.

It is also object of the present invention that of suggesting a prosthesis having mechanical and cinematic properties that can be compared with those of a human intervertebral disco, permitting to patients to carry out all possible movements with spine.

These objections are solved by an intervertebral disc prosthesis according to claim 1.

Always according to the invention, said prosthesis can comprise a first upper arm and a second upper arm, and a first lower arm and a second lower arm, said first upper arm and second upper arm and said firs lower arm and second lower arm being coupled each other so that they can take at least two positions, respectively with their longitudinal axes parallel and their longitudinal axes orthogonal.

Still according to the invention, in said opened operating position, the longitudinal axes of said first upper arm and of said second upper arm can form a substantially right angle, the longitudinal axes of said first lower arm and of said second lower arm form a substantially right angle, and the longitudinal axes of said first upper arm and of said second lower arm are substantially parallel.

Advantageously, according to the invention, said junction intermediate element can comprise a first body and a second body coupled by a spherical joint.

Always according to the invention, said first body has an upper surface capable to be housed in a circular seat of the arm, and a lower surface provided with a cavity, and said body has a upper convex surface, capable to be inserted in said cavity, by fixed joint or by compression coupling, in order to form a spherical joint, and a lower surface capable to be housed in a circular seat of the arm.

Still, according to the invention, said circular seat can comprise steps arranged circularly and having a progressively growing height, and each one of said upper surface of said first body and said lower surface of said second body comprises a projection capable of interacting with said steps of the respective circular seat.

Furthermore, according to the invention, said first and second body of said junction intermediate element each comprise a set of perimeter teeth which are arranged laterally.

Advantageously, according to the invention, said at least one upper arm and said at least one lower arm have a central hole, and said coupling means comprise two tightening nuts each one inserted in said holes, respectively of said at least one upper arm and of said lower arm, and coupled to said junction intermediate element.

Still according to the invention, each one of said tightening nuts has a substantially cross shaped opening and a helicoidal groove on the lateral surface, and in that it comprises a blocking pin including a couple of tabs, arranged substantially at 180° each other, which insert in the slots of said cross shaped opening and suitable to act on said helicoidal groove, so as to follow the rotation of said at least one upper and lower arm), said blocking pin comes out from said tightening nut.

It is further object of the present invention an intervertebral disc prosthesis kit, comprising at least one intervertebral disc prosthesis as defined in the above, and at least one insertion-extraction device comprising:
- a support element comprising one stem having a first end, on which a handle and a first and a second knob are provided, and a second end;
- a pliers comprising two jaws centrally pivoted in correspondence of a fulcrum having a hole, each one of said jaws comprising a handle, suitable to be arranged on said second end of said support element, and a grasp element, and being able to operate on one of the set of teeth of said junction intermediate element, said pliers further comprising a threaded bar rotatable by said first knob which passes through said hole and having a stop, said bar being engaged with a slide by a threaded hole; and
- a pair of hinged arms coupled by said central threaded hole of said slide to said threaded bar, an end of said hinged arms being pivoted in correspondence of said threaded hole, while the other end is pivoted close to said grasp element of said jaws.

Always according to the invention, each one of said hold elements of said jaws comprises a screw that can be activated by said second knob, suitable to operate with a respective perimeter set of teeth of said junction intermediate element.

Still according to the invention, said kit comprises an element for completing the thread suitable to complete the thread of said prosthesis, said thread being made by the semi-threads in said closed operating position.

The present invention will be now described, for illustrative but not limitative purposes, according to its preferred embodiments, with particular reference to the figure of the enclosed drawings, wherein:
figure 1 shows a perspective view of an embodiment of intervertebral disc prosthesis according to the invention in an open position;
figure 2 shows a top view of prosthesis of figure 1;
figure 3 is an exploded view of prosthesis of figure 1;
figure 4 shows a perspective view of prosthesis according to figure 1 in a closure position;
figure 5 shows an intermediate element of intervertebral disc prosthesis according to the invention;
figure 6 shows coupling with an arm of prosthesis of intermediate element of figure 5;
figure 7 shows an exploded view of a fixing nut with the relevant anchoring pin of prosthesis according to the invention;
figure 8 shows a lateral view of a intervertebral disc prosthesis according to the invention between two vertebral bodies;
figure 9 shows a further lateral view of a intervertebral disc prosthesis according to the invention between two vertebral bodies;
figure 10 shows a perspective view of a device for insertion-withdrawal of prosthesis according to the present invention;
figure 11 shows a particular of insertion-withdrawal device of figure 10;
figure 12 shows pliers of the insertion-withdrawal device of figure 10;
13 shows a particular of pliers of insertion-withdrawal device of figure 10;
figure 14 shows jaws of pliers of insertion-withdrawal device of figure 10;
figure 15a shows a first coupling step of a prosthesis according to the invention with pliers of the insertion-withdrawal device;
figure 15b shows a second coupling step of a prosthesis according to the invention with pliers of the insertion-withdrawal device;
figure 15c shows a third coupling step of a prosthesis according to the invention with pliers of the insertion-withdrawal device;
figure 15d shows a fourth coupling step of a prosthesis according to the invention with pliers of the insertion-withdrawal device;
figure 16 shows a plan view of the prosthesis in an operative position open by the insertion-withdrawal device;
figure 17 shows a perspective view of the prosthesis of figure 16; and
figure 18 shows a cross section of the prosthesis according to the invention.

Similar parts of the various figures will be indicated by the same reference numbers.

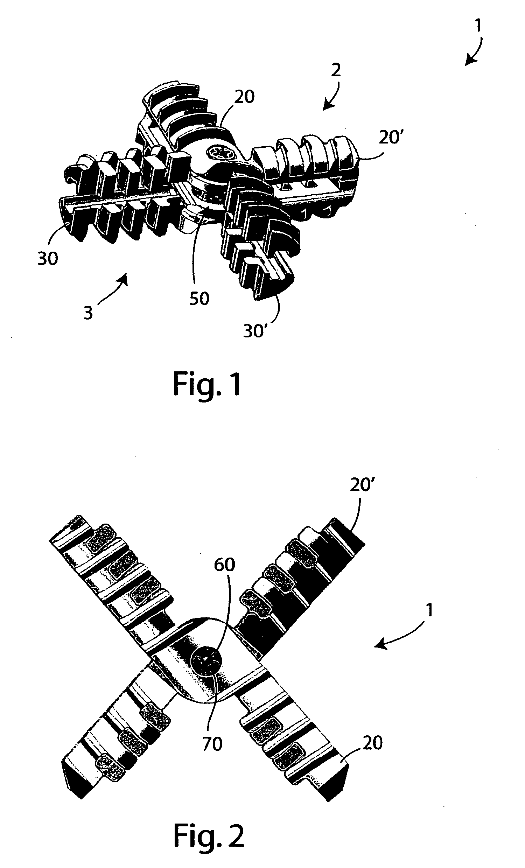

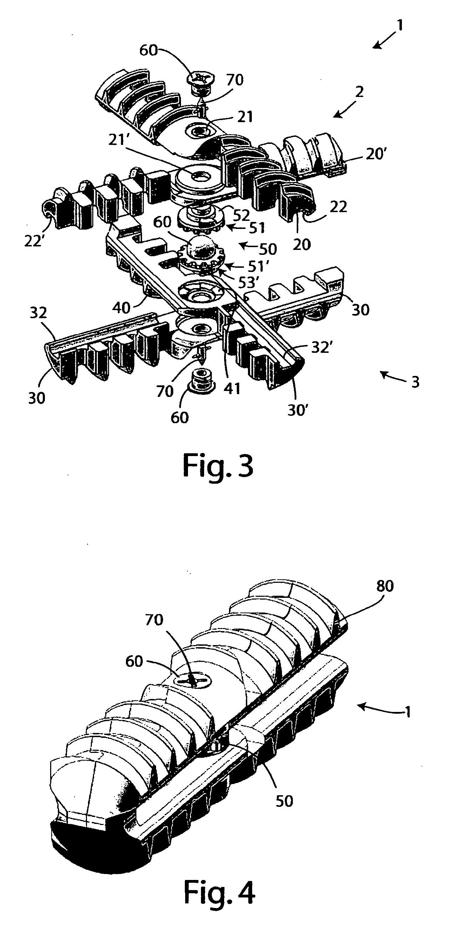

Making reference to figures 1 - 4, it is possible observing parts of an embodiment of intervertebral prosthesis 1 according to the invention.

As it can be observed, said prosthesis 1 comprises a junction intermediate element 50, to which a first couple of upper arms 2 and a second couple of lower arms 3. Each couple of upper 2 and lower 3 arms comprise a coupling arm 20', 30' with said junction intermediate element 50 and a second arm 20, 30. Arms 20, 20' and 30, 30' of each couple are juxtaposed each other and rotating one with respect to the other one about a central axis and realised in such a way to couple each other by graft, when parallel each other.

Each one of said arms 20, 20' and 30, 30' has a central hole, respectively holes 21, 21', 31, 31'.

About said holes 21' and 31' there are realised circular seats 40 (only one of which can be seen in the figures), wherein steps 41 are provided, provided along a circle and with a progressively growing height, the function of which will be better precised in the following.

Finally, each arm 20, 20', 30 and 30' has inner channels, respectively 22, 22', 23 and 23', the function of which will be better precised in the following.

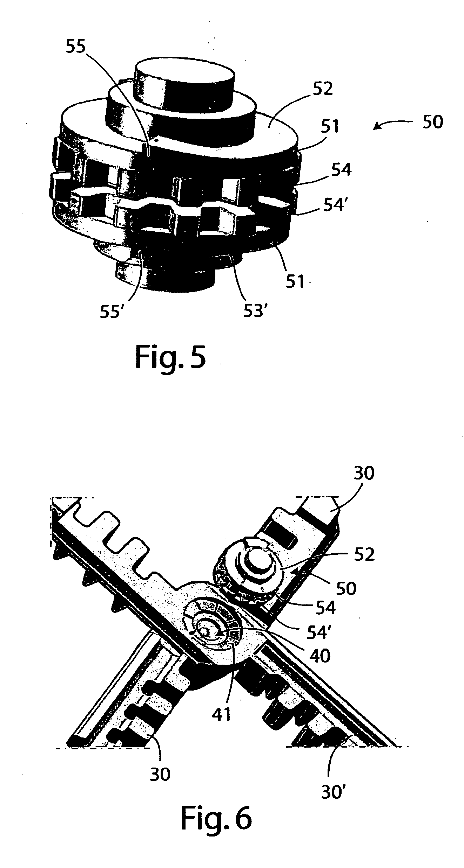

Said junction intermediate element 50 (see also figure 5) comprises a first body 51, having an upper surface 52 seating within the circular seat 40 of arm 20', and a lower surface provided with a cavity (not shown in the figures), and a second body 51', having a convex upper surface 52', suitable for inserting within said cavity, by a fixed joint or compression coupling, so as to create a spherical joint, and a lower surface 53', seating within the circular seat 40 of said arm 30'.

Making reference to figures 5 and 6, and analysing in greater detail junction intermediate element 50, it is observed that said first body 51 and said second body 51' laterally have each one perimeter teeth, respectively 54 and 54', so realised to permit coupling of a insertion/withdrawal device of prosthesis 1, that will be described in greater detail in the following.

Furthermore, said first and second bodies 51 and 51' comprise projections 55 and 55' on the relevant upper 52 and lower 53' surfaces, suitable for interacting with said steps 41 of said circular seats 40. Structure of the lower (convex) surface of body 51 and of upper (concave) surface 52' of body 51' permits the rotation each other (the aim of which will be clearer in the following of the present description). Steps 41 having a growing height and projections 55, 55' of bodies 51, 51' surfaces seating within seats 40 permits, following rotation each other of the two bodies 51, 51', modifying height of prosthesis 1.

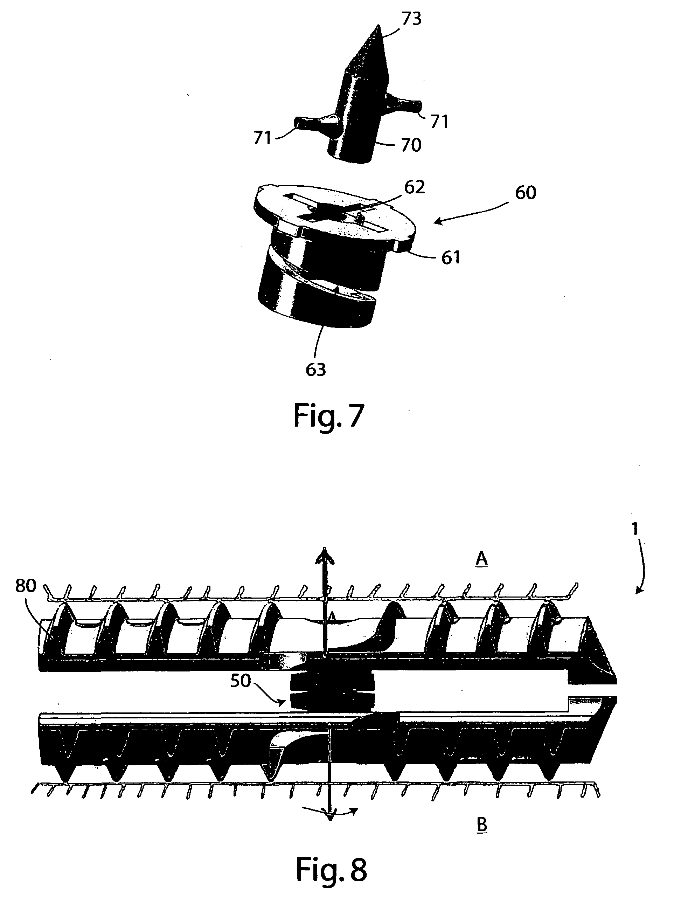

Two tightening nuts 60 couple, respectively, within holes 21, 21' and 31, 31' of arms 20, 20' and 30, 30', respectively. Each one of said nuts 60 have tabs 61 (see also figure 7) for coupling with arm, respectively arm 20 or 30, and, on the upper surface, a substantially cross - shaped slot for insertion of a relevant anchoring pin 70, having a pair of tabs 71, substantially at 180° each other, inserting within slots of said cross shaped opening 62.

Laterally with respect to said nut 60 it is realised an helicoidal groove 63, within which, after the insertion of pin 70, act on tabs 71, so that, following the rotation of arms 20, 20' and 30, 30', pin 70 exits from nut 60, and thus from prosthesis 1, thus obtaining an anchoring of vertebral bodies A and B by pin 70 tip 73.

Said prosthesis 1 can therefore take a closed operative position, wherein said arms 20, 20', 30 and 30' have their longitudinal axes offset, and particularly in the present embodiment, arms of each couple 2 or 3 have their axes forming a substantially 90° angle, as limit position.

Outer surface of arms 20, 20' and 30, 30' has a configuration with a semi thread 80 so as to realise, in the closed operative position of said prosthesis 1, a threading.

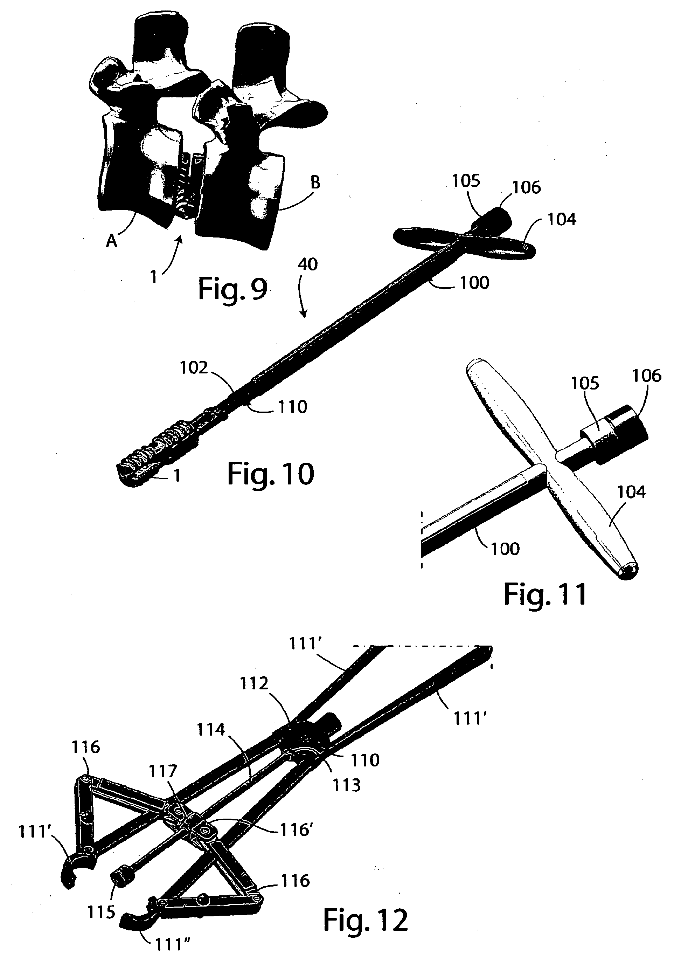

Figures 8 and 9 show prosthesis 1 placed between two vertebral bodies A and B, in an open position. Particularly, figure 8 shows how prosthesis 1 according to the invention can be stressed under flexion and compression (please note arrows of figure). Furthermore, fixing of prosthesis 1 to vertebral bodies is obtained by the action of semi threads realised on couples of arms 2 and 3, and by the lifting of anchoring pins 70 (described in detail in the above).

Particularly, said prosthesis 1 is inserted between vertebral bodies in a closed position. Once said prosthesis 1 is inserted by threading between the two vertebral bodies A and B and it is open, threading realised by semi threads 80 of arms 20, 20' and 30, 30' inserts within bone tissue of vertebral bodies A and B between which prosthesis is to be placed, ensuring primary fixing and obtaining a high resistance to retropulsion, thus permitting, in particular, an optimum restoring of the proper segmentary lumbar lordosis.

It must be taken into consideration that junction intermediate element 50 is comprised of a material able to ensure set mechanical features and to support loading conditions of the lumbar tract which is, as it is well known, subjected to different mechanical stresses by rotation and torsion, besides by flexion. Said material is also inert under a biological point of view, thus having a low rejection possibility.

Therefore, junction intermediate element 50, permits maintaining movement physiological range under flexion/extension, rotation and lateral inclination, as well as restoring of height of the relevant segment, of the anatomic balancing and of stability of spine.

Making now reference to figures 10 - 14, it is observed structure of insertion/withdrawal device 90 of said prosthesis 1 within/from a couple of vertebral bodies.

Said insertion/withdrawal device 90 comprises a support element 100 made up of a stem 101, having a first and a second end 102 and 103. A handle 104 and a first and second knob 105 and 106, juxtaposed each other, are provided on said first end 102.

Pliers 110 couple on end 103 of said stem 101. Said pliers 110 is comprised of two jaws 11, centrally pivoted in correspondence of fulcrum 112, having each one a handle 111' and a grip element 111". Said fulcrum 112 further has a hole 113 through which a rod 114 passes, with a stop end 115 acting as guide for a slide 116'. A couple of linked arms 116 is coupled with said slide 116'. One end of the linked arms 116 is pivoted in correspondence of said threaded hole 117 (i.e. slide 116'), while the other end is pivoted close to the grip element 111" of the jaws 111.

Two screws 118, acting each one on one of the teeth 54 and 54' of the intermediate element 50, are provided inside on grip elements 111", which are substantially arc shape.

When said insertion/withdrawal device 90 is coupled with said prosthesis 1, front parts of linked 116 arms inserts within inner channels 22, 22', 32 and 32' of said arms 20, 20', 30 and 30'. Thus, said mechanism is suitable to permit passage from said closed operative position to said open operative position of said prosthesis 1.

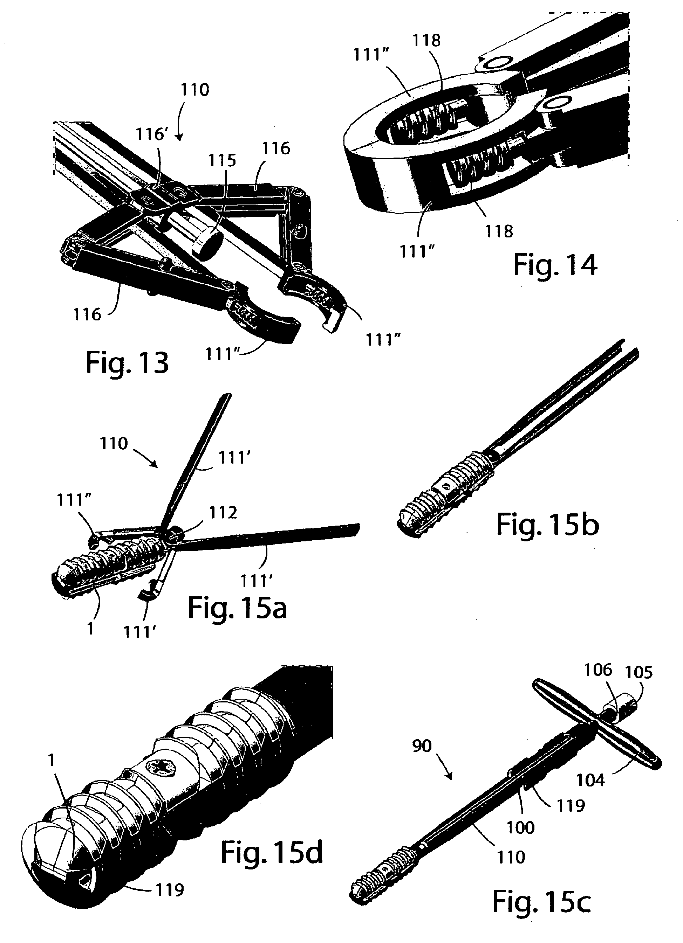

Observing figures 15 a - 15d, it is noted coupling of prosthesis 1 with pliers 110, occurring first coupling said ends 111' and 111" between the perimeter teeth 54 and 54'.

Preferably, prosthesis 1 also comprises an element completing threading 119 that can be inserted beforehand within insertion/withdrawal device 90, compensating shaped parts of arms 20, 20', 30 and 30'.

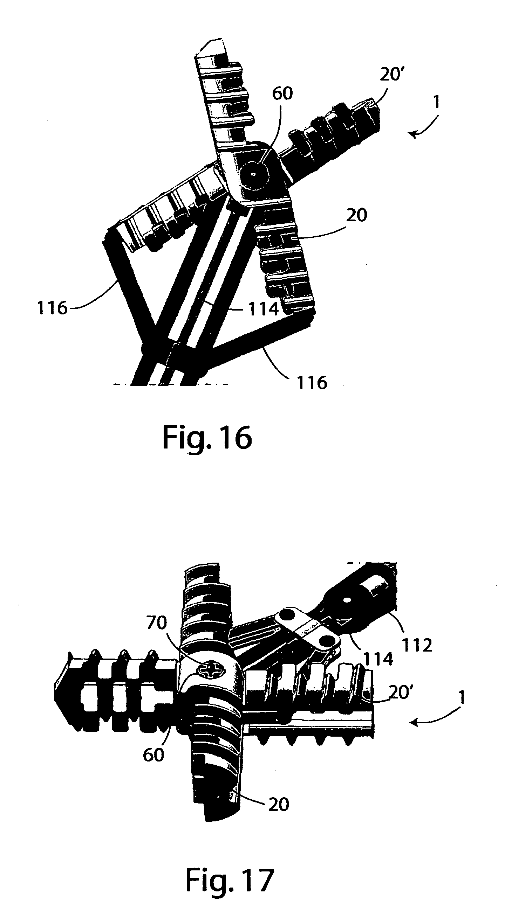

Once coupled pliers 110 with prosthesis 1, it is inserted the same between the two vertebral discs A and B. Thus, operator acts on knob 105, transmitting rotary movement by a hollow shaft (not shown in the figures) passing within the support element 100. Said hollow shaft couples with rod 114, which is threaded thus permitting sliding of slide 116' and thus opening movement of prosthesis 1 arms, said prosthesis 1 acting on pushing rod 114 causing, by the action of linked arms 116, rotation of couples of arms 2 and 3 with respect to intermediate element 50, as it can be observed from figures 16 and 17.

At the same time, tip 73 of anchoring pin 70 lifts, anchoring with vertebral bodies A and B.

Screws 108, while pliers 110 is still closed and tightens intermediate element 50 by grip elements 101', are activated by said knob 96. Knob 106 transmits rotary movement by a further shaft (not shown in the figures) which is concentric with respect to said hollow shaft passing through the pliers 110 body. Said further shaft is provided, on its other end, with a toothed wheel (not shown in the figure) operating inside the grip elements 111' and 111" (see figure 13). Said couple of secondary shafts which are inside the grip elements 111' and 111" couple, on the other end, with screws 118 (please see figure 14).

Said screws 108 rotating act each one on said perimeter teeth, respectively 54 and 54', increasing height of prosthesis 1.

Then, it is sufficient withdrawing support element 90 from handles 94 of pliers 100, opening pliers 100 widening jaws 101 and decouple said pliers 100 from said open prosthesis 1.

Surgical technique that can be used by the present prosthesis 1 is carried out by a general anaesthesia. When prosthesis 1 according to the invention is in a closed position, it takes a longitudinal cylindrical shape with a lateral threading, thus resembling a screw. This permits introducing the same from behind, by a discectomy.

In order to implant said prosthesis 1, the following steps are particularly carried out:
- placing prosthesis 1 in an operative closure position;
- coupling prosthesis 1 with said insertion/withdrawal device 90;
- carrying out a discectomy and a posterior laminotomy, i.e. rear vertebral arc is open, by a mini-invasive opening, in order to contain diameter of intervertebral prosthesis 1;
- screwing prosthesis 1 only within sub-condral bone portion and not in the spongy portion, by said insertion/withdrawal device 90; and
- opening or expanding prosthesis 1 and removing said insertion/withdrawal device 90.

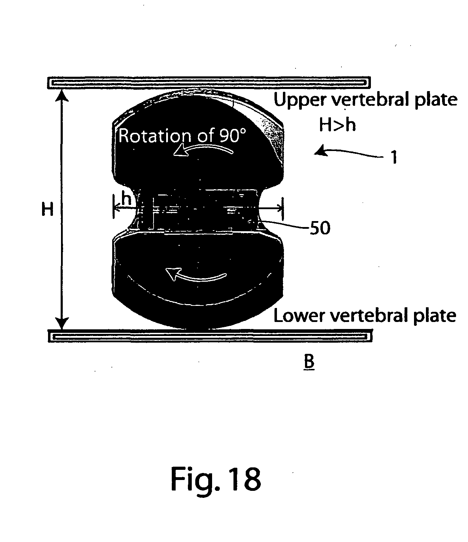

Removal of prosthesis 1 is very easy thanks to its cross section that can be observed in figure 18. When it is necessary removing the prosthesis 1, it is sufficient coupling prosthesis 1 with pliers 100, making the threading 119 fully descending, and rotating pliers about its own longitudinal axis, withdrawing the same from vertebral bodies.

Different embodiments of intervertebral prosthesis 1 are possible that can also provide a different number of upper arms, even only one, and a different number of lower arms, in this case too even only one.

An advantage of the present invention is that of permitting its implantation from behind, carrying out a discectomy and a posterior laminotomy on patient, i.e. a well consolidated surgical procedure not introducing further surgical risks.

Another advantage of the present invention is that the intermediate element guarantees a movement range very close to the one characterising each level of the spine lumbar column (Yamamoto).

A further advantage of the present invention is that it permits respecting anatomy of neuronal arc in order to maintain a higher stability of the segment subjected to treatment, thanks to the fact that intersomatic prosthesis permits, thanks to its shape and characteristic of accession from behind and mini-invasive, as well as thanks to preservation of the functional part of articolar faces. Its design permits an easy insertion, while cylindrical body, threading and fixing pins ensures an optimum stability guaranteeing primary bone fixation and preventing migration.

Another advantage of the invention is that prosthesis is provided with a mechanism permitting varying its height.

Furthermore, artificial disc must permit normal movements of spine so that the same (artificial disc) shapes on the basis of vertebrae movement. Furthermore, prosthesis is suitable to support both normal and maximum loading conditions, operates as stand-alone device if rear elements are integral, resisting to wearing and permits osteo-integration to the bone-prosthesis interface.

A further advantage of the present invention is that prosthesis permits restoring a rotation centre closer to the physiological one with respect to those with fixed instantaneous rotation centre (CIR) and transferring a reduced stress on articular faces. For unconstrained prosthesis as the one under examination, it is very important the role of soft tissues in order to guarantee stability, maintaining as more as possible posterior longitudi nal ligament (PLL) so as to increase stability of the implant, thus limiting hyper flexion forces. Furthermore, unconstrained prosthesis much more tolerate positioning slightly displaced from the optimum one, forgiving the surgeon, when his making his first field prosthetic experiences, limited positioning mistaken.

## Claims

1. Intervertebral disc prosthesis (1) interposable-between two vertebral bodies (A, B), comprising
at least one upper arm (20, 20') extending essentially longitudinally,
at least one lower arm (30, 30') extending essentially longitudinally, and
a junction intermediate element (50), interposed between said at least one upper arm (20, 20') and said at least one lower arm (30, 30'), and rotatably coupling said arms by coupling means (60),
said prosthesis (1) taking a closed operating position, in which said arms (20, 20', 30, 30') are arranged with the longitudinal axes substantially aligned, and an opened operating position, in which said arms (20, 20', 30, 30') have the axes out of alignment.
**characterized in that**
the external surface of said arms (20, 20', 30, 30') has a semi thread (80), such that it constitutes a complete thread in said closed operating position, and such that in said opened operating position said semi-threads (80) are engaged to the vertebral bodies (A, B), between which said prosthesis (1) is interposable.

2. Prosthesis (1) according to claim 1, **characterized in that** it comprises a first upper arm (20) and a second upper arm (20'), and a first lower arm (30) and a second lower arm (30'), said first upper arm and second upper arm and said firs lower arm and second lower arm being coupled each other so that they can take at least two positions, respectively with their longitudinal axes parallel and their longitudinal axes orthogonal.

3. Prosthesis (1) according to claim 2, **characterized in that** in said opened operating position, the longitudinal axes of said first upper arm (20) and of said second upper arm (20') form a substantially right angle, the longitudinal axes of said first lower arm (30) and of said second lower arm (30') form a substantially right angle, and the longitudinal axes of said first upper arm (20) and of said second lower arm (30') are substantially parallel.

4. Prosthesis (1) according to anyone of the preceding claims, **characterized in that** said junction intermediate element (50) comprises a first body (51) and a second body (51') coupled by a spherical joint.

5. Prosthesis (1) according to claim 4, **characterized in that** said first body (51) has an upper surface (52) capable to be housed in a circular seat (40) of the arm (20'), and a lower surface provided with a cavity, and said second body has a upper convex surface (52'), capable to be inserted in said cavity, by fixed joint or by compression coupling, in order to form a spherical joint, and a lower surface (53') capable to be housed in a circular seat (40) of the arm (30').

6. Prosthesis (1) according to claim 5, **characterized in that** said circular seat (40) comprises steps (41) arranged circularly and having a progressively growing height, and each one of said upper surface (52) of said first body (51) and said lower surface (53') of said second body (51) comprises a projection (55, 55') capable of interacting with said steps (41) of the respective circular seat (40).

7. Prosthesis (1) according to anyone of the claims 4 - 6, **characterized in that** said first (51) and second (51') body of said junction intermediate element (50) each comprise a set of perimeter teeth (54, 54') which are arranged laterally.

8. Prosthesis (1) according to anyone of the preceding claims, **characterized in that** said at least one upper arm (20, 20') and said at least one lower arm (30, 30') have a central hole (21, 21', 31, 31'), and said coupling means comprise two tightening nuts (60) each one inserted in said holes (21, 21', 31, 31'), respectively of said at least one upper arm (20, 20') and of said lower arm (30, 30'), and coupled to said junction intermediate element (50).

9. Prosthesis (1) according to claim 8, **characterized in that** each one of said tightening nuts (60) has a substantially cross shaped opening (62) and a helicoidal groove (63) on the lateral surface, and **in that** it comprises a blocking pin (70) including a couple of tabs (71), arranged substantially at 180° each other, which insert in the slots of said cross shaped opening (62) and suitable to act on said helicoidal groove (63), so as to follow the rotation of said at least one upper and lower arm (20, 20', 30, 30'), said blocking pin (70) comes out from said tightening nut (60).

10. Intervertebral disc prosthesis kit, comprising at least one intervertebral disc prosthesis (1) as defined in claims 1-9, and at least one insertion-extraction device (90) comprising:
- a support element (100) comprising one stem (101) having a first end (102), on which can handle and a first and a second knob (105, 106) are provided, and a second end (103);
- a pliers (110) comprising two jaws (111) centrally pivoted in correspondence of a fulcrum (112) having a hole (113), each one of said jaw (111) comprising a handle (111'), suitable to be arranged on said second end (103) of said support element (100), and a grasp element (111"), and being able to operate on one of the set of teeth (54, 54') of said junction intermediate element (50), said pliers (110) further comprising a threaded bar (114) rotatable by said first knob (105), which passes through said hole (113) and having a stop (115), said bar (114) being engaged with a slide (116') by a threaded hole (117); and
- a pair of hinged arms (116) coupled by said central threaded hole (117) of said slide (116') to said threaded bar (114), an end of said hinged arms (116) being pivoted in correspondence of said threaded hole (117), while the other end is pivoted close to said grasp element (111") of said jaws (111).

11. Kit according to claim 10, **characterized in that** each one of said hold elements (111") of said jaws (111) comprises a screw (118), that can be activated by said second knob (106), suitable to operate with a respective perimeter set of teeth (54, 54') of said junction intermediate element (50).

12. Kit according to anyone of the claims 10 - 11, **characterized in that** it comprises an element for completing the thread (119) suitable to complete the thread of said prosthesis (1), said thread being made by the semi-threads (80) in said closed operating position.

## Patentansprüche

1. Bandscheidenprothese (1), die zwischen zwei Wirbelkörper (A, B) eingefügt werden kann, umfassend
mindestens einen oberen Arm (20, 20'), der sich im Wesentlichen in Längsrichtung erstreckt,
mindestens einen unteren Arm (30, 30'), der sich im Wesentlichen in Längsrichtung erstreckt, und
ein Kreuzungszwischenelement (50), das zwischen den mindestens einen oberen Arm (20, 20') und den mindestens einen unteren Arm (30, 30') eingefügt ist und die Arme durch Verbindungsmittel (60) drehbar miteinander verbindet,
wobei die Prothese (1) eine geschlossene Betriebsstellung einnimmt, in der die Arme (20, 20', 30, 30') mit den Längsachsen im Wesentlichen ausgerichtet sind, und eine geöffnete Betriebsstellung, in der die Arme (20, 20', 30, 30') die Achsen nicht ausgerichtet haben,
**dadurch gekennzeichnet, dass**
die Außenfläche der Arme (20, 20', 30, 30') ein Halbgewinde (80) aufweist, sodass sie in der geschlossenen Betriebsstellung ein komplettes Gewinde bildet, und dass die Halbgewinde (80) in der geöffneten Betriebsstellung im Eingriff stehen mit den Wirbelkörpern (A, B), zwischen die die Prothese (1) eingefügt werden kann.

2. Prothese (1) gemäß Anspruch 1, **dadurch gekennzeichnet, dass** sie einen ersten oberen Arm (20) und einen zweiten oberen Arm (20') und einen ersten unteren Arm (30) und einen zweiten unteren Arm (30') aufweist, wobei der erste obere Arm und der zweite obere Arm und der erste untere Arm und der zweite untere Arm miteinander so verbunden sind, dass sie mindestens zwei Positionen einnehmen können mit ihren Längsachsen parallel oder ihren Längsachsen rechtwinklig.

3. Prothese (1) gemäß Anspruch 2, **dadurch gekennzeichnet, dass** in der geöffneten Betriebsstellung die Längsachsen des ersten oberen Arms (20) und des zweiten oberen Arms (20') einen im Wesentlichen rechten Winkel bilden, die Längsachsen des ersten unteren Arms (30) und des zweiten unteren Arms (30') einen im Wesentlichen rechten Winkel bilden und die Längsachsen des ersten oberen Arms (20) und des zweiten unteren Arms (30') im Wesentlichen parallel sind.

4. Prothese (1) gemäß einem der vorangehenden Ansprüchen, **dadurch gekennzeichnet, dass** das Kreuzungszwischenelement (50) einen ersten Körper (51) und einen zweiten Körper (51') aufweist, die durch ein Kugelgelenk verbunden sind.

5. Prothese (1) gemäß Anspruch 4, **dadurch gekennzeichnet, dass** der erste Körper (51) eine obere Fläche (52) aufweist, die in der Lage ist, um von einem kreisförmigen Sitz (40) des Arms (20') aufgenommen zu werden, und eine untere Oberfläche mit einem Hohlraum versehen ist, und der zweite Körper eine obere konvexe Oberfläche (52') aufweist, die in der Lage ist, um in den Hohlraum eingesetzt zu werden durch eine feste Verbindung oder eine Klemmkupplung, um ein Kugelgelenk zu bilden, und eine Unteroberfläche (53), die in der Lage ist, in einem kreisförmigen Sitz (40) des Arms (30') aufgenommen zu werden.

6. Prothese (1) gemäß Anspruch 5, **dadurch gekennzeichnet, dass** der kreisförmige Sitz (40) Stufen (41) aufweist, die kreisförmig angeordnet sind und eine progressiv anwachsende Höhe aufweisen, und jeder der oberen Oberfläche (52) des ersten Körpers (51) und der unteren Oberfläche (53') des zweiten Körpers (51) einen Vorsprung (50, 55') aufweist, der in der Lage ist, um mit den Stufen (41) des jeweiligen kreisförmigen Sitzes (40) zusammenzuwirken.

7. Prothese (1) gemäß einem der Ansprüche 4-6, **dadurch gekennzeichnet, dass** der erste (51) und der zweite (51') Körper des Kreuzungszwischenelements (50) jeweils einen Satz von Umfangszähnen (54, 54') aufweist, die seitlich angeordnet sind.

8. Prothese gemäß einem der vorangehenden Ansprüchen, **dadurch gekennzeichnet, dass** der mindestens eine obere Arm (20, 20') und der mindestens eine untere Arm (30, 30') eine Mittelbohrung (21, 21', 31, 31') aufweist und das Verbindungsmittel zwei Befestigungsmuttern (60) aufweist, von denen jede in eine der Bohrungen (21, 21', 31, 31') des mindestens einen oberen Arms (20, 20') bzw. des unteren Arms (30, 30') eingesetzt ist und mit dem Kreuzungszwischenelement (50) verbunden ist.

9. Prothese (1) gemäß Anspruch 8, **dadurch gekennzeichnet, dass** jede der Befestigungsmuttern (60) eine im Wesentlichen kreuzförmige Öffnung (62) und eine Spindelnut (63) an der seitlichen Oberfläche aufweist und dass sie einen Blockierstift (70) aufweist, der ein paar Tabs (71) enthält, die im Wesentlichen mit 180° zueinander angeordnet sind, die in die Schlitze der kreuzförmigen Öffnungen (62) einsetzen und geeignet sind, um auf die Spindelnut (63) zu wirken, um der Rotation des mindestens einen oberen und unteren Arms (20, 20', 30, 30') zu folgen, wobei der Blockierstift (70) aus der Befestigungsmutter (60) herauskommt.

10. Bandscheibenprothesensatz, umfassend mindestens eine Bandscheibenprothese (1), wie sie in den Ansprüchen 1-9 definiert ist, und mindestens ein Einführ-Entnahmegerät (90) umfassend:
- ein Halteelement (100) mit einem Schaft (101) mit einem ersten Ende (102), an dem man handhaben kann und an dem ein erster und ein zweiter Griff (105, 106) vorgesehen ist, und einem zweiten Ende (103);
- eine Zange (110) umfassend zwei Backen (111), die mittig entsprechend einer Drehachse (112) mit einem Loch (113) schwenkbar gelagert sind, wobei jede der Backen (111) einen Griff (111') aufweist, der geeignet auf dem zweiten Ende (103) des Halteelements (100) angeordnet ist, und ein Griffelement (111''), und die geeignet ist, um auf einen der Zahnsätze (54, 54') des Kreuzungszwischenelements (50) zu arbeiten, wobei die Zange (110) weiterhin eine mit einem Gewinde versehene Stange (114) aufweist, die durch den ersten Griff (105) drehbar ist, die durch das Loch (113) hindurch verläuft und einen Anschlag (115) aufweist, wobei die Stange (114) in einen Schlitten (116') durch ein mit einem Gewinde versehenes Loch (117) eingreift;
- ein paar von angewinkelten Armen (116), die durch das mit einem Gewinde versehene mittige Loch (117) des Schlittens (116') mit der mit einem Gewinde versehenen Stange (114) verbunden sind, wobei ein Ende der angewinkelten Arme (116) entsprechend dem mit einem Gewinde versehenen Loch (117) geschwenkt wird, während das andere Ende nahe an das Griffelement (111'') der Backen (111) geschwenkt wird.

11. Satz gemäß Anspruch 10, **dadurch gekennzeichnet, dass** jedes der Halteelemente (111'') der Backen (111) eine Schraube (118) aufweist, die von dem zweiten Griff (106) aktiviert werden kann, die geeignet ist, um mit einem jeweiligen Umfangszahnsatz (54, 54') des Kreuzungszwischenelements (50) zu wirken.

12. Satz gemäß einem der Ansprüche 10-11, **dadurch gekennzeichnet, dass** er ein Element aufweist zur Vervollständigung des Gewindes (119), das geeignet ist, um das Gewinde der Prothese (1) zu vervollständigen, wobei das Gewinde in der geschlossenen Betriebsstellung von den Halbgewinden (80) gebildet wird.

## Revendications

1. Prothèse de disque intervertébral (1) pouvant être interposée entre deux corps vertébraux (A, B), comprenant
au moins un bras supérieur (20, 20') s'étendant sensiblement longitudinalement,
au moins un bras inférieur (30, 30') s'étendant sensiblement longitudinalement, et
un élément de jonction intermédiaire (50) interposé entre ledit au moins un bras supérieur (20, 20') et ledit au moins un bras inférieur (30, 30') et qui accouple lesdits bras avec possibilité de rotation par des moyens d'accouplement (60),
ladite prothèse (1) prenant une position de travail fermée dans laquelle lesdits bras (20, 20', 30, 30') sont disposés de telle manière que leurs axes longitudinaux soient sensiblement alignés et une position de travail ouverte dans laquelle lesdits bras (20, 20', 30, 30') ont leurs axes hors d'alignement.
**caractérisée en ce que**
la surface externe desdits bras (20, 20', 30, 30') présente un demi-filet (80), de sorte qu'elle constitue un filet complet dans ladite position de travail fermée, et de sorte que, dans ladite de position de travail ouverte, lesdits demi-filets (80) sont en contact avec les corps vertébraux (A, B) entre lesquels ladite prothèse (1) peut être interposée.

2. Prothèse (1) selon la revendication 1, **caractérisée en ce qu'**elle comprend un premier bras supérieur (20) et un second bras supérieur (20') ainsi qu'un premier bras inférieur (30) et un second bras inférieur (30'), lesdits premier bras supérieur et second bras supérieur ainsi que lesdits premier bras inférieur et second bras inférieur étant accouplés l'un à l'autre de telle manière qu'ils puissent prendre au moins deux positions, respectivement, une dans laquelle leurs axes longitudinaux sont parallèles et une dans laquelle leurs axes longitudinaux sont orthogonaux.

3. Prothèse (1) selon la revendication 2, **caractérisée en ce que**, dans ladite position de travail ouverte, les axes longitudinaux dudit premier bras supérieur (20) et dudit second bras supérieur (20') forment un angle sensiblement droit, les axes longitudinaux dudit premier bras inférieur (30) et dudit second bras inférieur (30') forment un angle sensiblement droit, et les axes longitudinaux dudit premier bras supérieur (20) et dudit second bras inférieur (30') sont sensiblement parallèles.

4. Prothèse (1) selon une quelconque des revendications précédentes, **caractérisée en ce que** ledit élément de jonction intermédiaire (50) comprend un premier corps (51) et un second corps (51') accouplés par un joint à rotule.

5. Prothèse (1) selon la revendication 4, **caractérisée en ce que** ledit premier corps (51) possède une surface supérieure (52) qui peut être logée dans une portée circulaire (40) du bras (20'), et une surface inférieure munie d'une cavité, et ledit second corps présente une surface supérieure convexe (52') qui peut être insérée dans ladite cavité, en joint bloqué ou en accouplement à compression, de manière à former un joint à rotule, et une surface inférieure (53') qui peut être logée dans une portée circulaire (40) du bras (30').

6. Prothèse (1) selon la revendication 5, **caractérisée en ce que** ladite portée circulaire (40) comprend des gradins (41) arrangés en une disposition circulaire et présentant une hauteur progressivement croissante, et chaque surface de ladite surface supérieure (52) dudit premier corps (51) aussi bien que ladite surface inférieure (53') dudit second corps (51), comprend une saillie (55, 55') capable d'interagir avec lesdits gradins (41) de la portée circulaire (40) respective.

7. Prothèse (1) selon une quelconque des revendications 4 à 6, **caractérisée en ce que** ledit premier corps (51) et ledit second corps (51') dudit élément de jonction intermédiaire (50) comprennent chacun un jeu de dents périphériques (54, 54') qui sont disposées latéralement.

8. Prothèse (1) selon une quelconque des revendications précédentes, **caractérisée en ce que** ledit au moins un bras supérieur (20, 20') et ledit au moins un bras inférieur (30, 30') présentent un trou central (21, 21', 31, 31') et lesdits moyens d'accouplement comprennent deux écrous de serrage (60), engagés respectivement dans lesdits trous (21, 21', 31, 31') dudit au moins un bras supérieur (20, 20') et dudit au moins un bras inférieur (30, 30') et qui sont accouplés au dit élément de jonction intermédiaire (50).

9. Prothèse (1) selon la revendication 8, **caractérisée en ce que** chacun desdits écrous de serrage (60) présente une ouverture (62) sensiblement en forme de croix et une gorge hélicoïdale (63) sur la surface latérale, et **en ce qu'**il comprend une cheville de blocage (70) comprenant deux pattes (71), disposées sensiblement à 180° l'une de l'autre, qui s'engagent dans les fentes de ladite ouverture en forme de croix (62) et sont appropriées pour agir sur ladite gorge hélicoïdale (63) de manière à suivre la rotation dudit au moins un bras supérieur et bras inférieur (20, 20', 30, 30'), ladite goupille de blocage (70) sortant dudit écrou de serrage (60).

10. Ensemble d'éléments de prothèse de disque intervertébral, comprenant au moins une prothèse de disque intervertébral (1) selon les revendications 1 à 9 et au moins un dispositif d'insertion-extraction (90), qui comprend :
- un élément support (100) comprenant une tige (101) qui possède une première extrémité (102) sur laquelle sont prévues une poignée et des première et seconde molettes (105, 106), et une seconde extrémité (103) ;
- une pince (110) comprenant deux mâchoires (111) qui sont articulées au centre au niveau d'un pivot (112) qui présente un trou (113), chacune desdites mâchoires (111) comprenant une poignée (111'), appropriée pour être agencée sur ladite seconde extrémité (103) dudit élément support (100), et un élément de serrage (111 "), et pouvant opérer sur l'un des jeux de dents (54, 54') dudit élément de jonction intermédiaire (50), ladite pince (110) comprenant en outre une barre filetée (114) qui peut être entraînée en rotation par ladite première molette (105), qui passe à travers ledit trou (113), et présente une butée (115), ladite barre (114) étant en prise avec un curseur (116') par un trou fileté (117), et
- deux bras articulés (116) accouplés à ladite barre filetée (114) par ledit trou fileté central (117) dudit curseur (116'), une extrémité desdits bras articulées (116) s'articulant au droit dudit trou fileté (117), tandis que l'autre extrémité s'articule à proximité dudit élément de serrage (111 ") desdites mâchoires (111).

11. Ensemble d'éléments selon la revendication 10, **caractérisé en ce que** chacun desdits des éléments de retenue (111") desdites mâchoires (111) comprend une vis (118) qui peut être actionnée par ladite seconde molette (106), et qui est appropriée pour agir sur un jeu de dents périphérique respectif (54, 54') dudit élément de jonction intermédiaire (50).

12. Ensemble d'éléments selon une quelconque des revendications 10 à 11, **caractérisé en ce qu'**il comprend un élément pour compléter le filet (119) approprié pour compléter le filet de ladite prothèse (1), ledit filet étant composé des demi-filets (80) dans ladite position de travail fermée.
